# EUROPÄISCHE PATENTSCHRIFT

(11) **EP 0 593 082 B1**
(45) Veröffentlichungstag und Bekanntmachung des Hinweises auf die Patenterteilung: **22.04.1998**
(21) Anmeldenummer: 93116734.0
(22) Anmeldetag: 15.10.1993
(51) Int. Cl.: A61F 13/15

(54) **Einwegwindeln**
Disposable diapers
Couches culottes à jeter

(30) Priorität: 15.10.1992 JP 72008/92
(43) Veröffentlichungstag der Anmeldung: 20.04.1994
(73) Patentinhaber: UNI-CHARM CORPORATION, Kawanoe-shi Ehime-ken (JP)
(72) Erfinder: Kido, Tsutomu, Kawanoe-shi, Ehime-ken (JP)
(74) Vertreter: Sperling, Rüdiger, Dipl.-Ing.

(56) Entgegenhaltungen:
- EP-A- 0 219 326
- EP-A- 0 243 013
- EP-A- 0 251 332
- US-A- 4 904 251

## Beschreibung

Die Erfindung betrifft Einwegwindeln gemäß dem Oberbegriff

des Anspruchs 1. Es ist wohl bekannt, Einwegwindeln mit Seitenlaschen auszustatten, die sich zwischen in Längsrichtung gegenüberliegenden Enden solcher Windein erstrecken und geeignet sind, auf der Oberseite solcher Windein emporzuragen, wenn die Windein einem Träger umgelegt werden, so daß eine Menge Körperflüssigkeiten wirkungsvoll daran gehindert werden kann, durch Beinöffnungen solcher Windein auszutreten.

Eine solche Technik ist z.B. in der Beschreibung der US-A-4 704 116 offenbart, gemäß der eine flüssigkeitsdurchlässige Oberschicht teilweise so gefaltet ist, daß sie ärmelartige Laschen bildet, die sich zwischen den in Längsrichtung gegenüberliegenden Enden einer Windel erstrecken und elastische Elemente unter Spannung mit diesen ärmelartigen Laschen in Längsrichtung verbunden sind, so daß diese ärmelartigen Laschen auf der Oberseite der Windel emporragen können, wenn sie einem Träger umgelegt wird. Eine derartige Technik ist auch aus den japanischen Patentanmeldungen No. 63 105102 und 63 112703 bekannt, gemäß welchen ein Paar von ärmelartigen Elementen, von denen jedes eine der in Längsrichtung einer Windel entsprechende Länge aufweist, getrennt angefertigt ist, und Laschen durch Verbinden/Verkleben dieser ärmelartigen Elemente mit der Oberschicht der Windel ausgebildet werden. Wenn die ärmelartigen Laschen oder Elemente aus einer flüssigkeitsdurchlässigen Schicht, wie die Oberschicht, gebildet sind, werden sie manchmal behandelt, um eine gewünschte Wasser-Abstoßfähigkeit Zu erhalten, um Körperflüssigkeiten am Durchsickern durch diese zu hindern.

Die zum Emporragen an der Oberseite der Windel ausgebildet Laschen sind, wenn die Windel einem Träger umgelegt wird, in der Lage, in der erwarteten Weise zu wirken, sofern sich die Laschen zumindest entlang der Längsausdehnung einer Schrittzone erstrecken. Dennoch erstrecken sich die ärmelartigen Laschen oder Elemente gemäß dem oben beschriebenen Stand der Technik vollständig zwischen den sich in Längsrichtung gegenüberliegenden Enden, d.h., die gegenüberliegenden Taillenlinien der Windel und zu den gegenüberliegenden Taillenlinien benachbart liegende Abschnitte dieser ärmelartigen Laschen sind geknickt und an/mit der Vorderschicht befestigt/verklebt. Eine solche Anordnung hat offensichtlich den Nachteil, daß die zu der Vorder- und Rücktaillenlinie benachbarten Zonen sperrig und steif werden, wodurch sich der Träger unwohl fühlt.

Demnach ist es eine wesentliche Aufgabe der Erfindung, das oben erwähnte Problem zu lösen, und zwar ohne einen Verlust in der Körperflüssigkeits-Dämmwirkung der Laschen durch Faltabschnitte von Ober- und/oder Unterschichten, die sich von transversal gegenüberliegenden Seiten eines absorbierenden Windelkernes nach außen erstrecken, um die Laschen so auszubilden, daß sie auf der Oberseite der Windel emporragen können, wenn sie einem Träger umgelegt wird, so daß die Laschen sich nicht über ein notwendiges Ausmaß, das im wesentlichen der Längsausdehnung der Schrittzone entspricht, ausdehnen.

Die oben beschriebene Aufgabe wird gemäß der Erfindung durch die Merkmale des kennzeichnenden Teils des Anspruchs 1 gelöst.

Mit der Windel dieser Anordnung bilden die Abschnitte der Ober- und/oder der Unterschicht, welche längs ihrem in Längsrichtung verlaufenden Maß innerhalb des Schrittbereichs befestigt sind, Laschen auf der Oberseite der Windel in einem Maß, das durch die in Längsrichtung verlaufenden sich gegenüberstehenden Enden bestimmt ist, wenn sich diese elastischen Teile zusammenziehen. Die Höhen der Laschen nehmen von dem Maximum in der Mitte der Schrittzone zu den in Langsrichtung gegenüberliegenden Enden nach und nach ab.

Die Einwegwindel gemäß der Erfindung wird leichter und genauer verstanden aufgrund der folgenden Beschreibung anhand von Beispielen im Zusammenhang mit den beiliegenden Zeichnungen, in welchen:
Fig. 1 eine perspektivische Ansicht einer gemäß der Erfindung ausgebildeten Einwegwindel ist;
Fig. 2 eine Schnittdarstellung entlang einer Linie A-A in Fig. 1 ist; und
Fig. 3A und 3B Ansichten ähnlich der Fig. 2 sind, die alternative Ausführungsbeispiele zweiter Laschen zeigen.

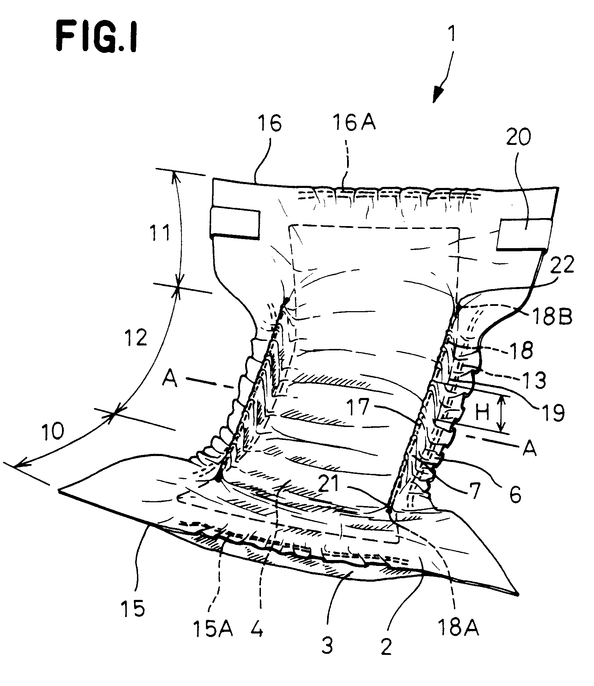

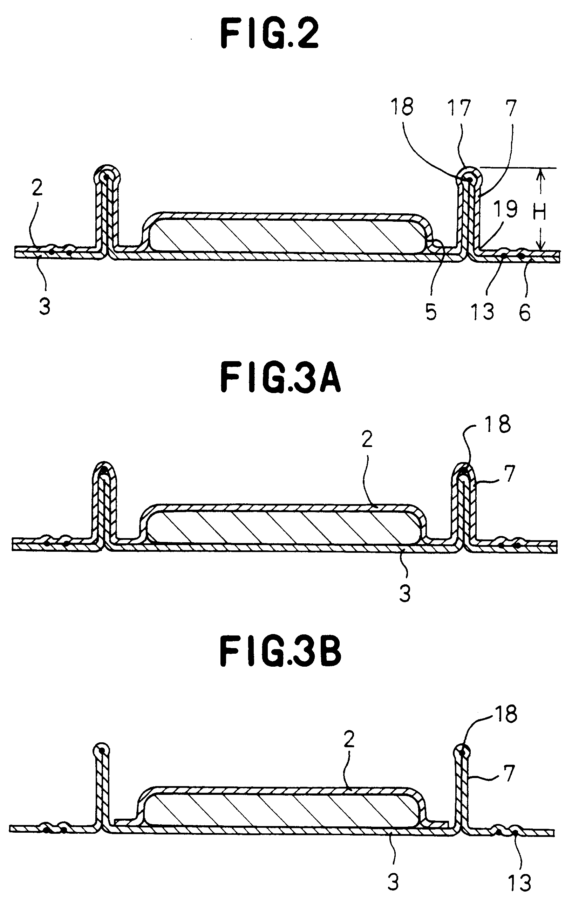

Fig. 1 und 2 stellen eine Einwegwindel 1 in einer perspektivischen Ansicht und in einer Schnittdarstellung entlang einer Linie A-A in Fig. 1 dar, und Fig. 1 veranschaulicht speziell einen Zustand, in dem die Windel 1 auf der Oberseite aufgrund von Kontraktion der elastischen Teile 13, 18 gekrümmt ist, wie später beschrieben wird. Die Windel 1 umfaßt im wesentlichen eine flüssigkeitsdurchlässige Oberschicht 2, eine flüssigkeitsundurchlässige Unterschicht 3 und einen absorbierenden, zwischen diesen Schichten 2, 3 eingelegten Kern 4. Die Abschnitte der Ober- und Unterschichten 2, 3, die sich über die transversal gegenüberliegenden Ränder 5 des absorbierenden Kerns 4 erstrecken, bilden erste, sich horizontal um jeweilige Beinöffnungen erstreckende Laschen 6 und zweite innerhalb der jeweiligen ersten Laschen 6 und zum Emporragen auf der Windeloberseite 1 geeignete zweite Laschen 7. Die Windel 1 ist in Längsrichtung aus Vorder- und Rückenteil 10, 11 und einem sich zwischen diesem Vorder- und Rückenteil 10, 11 erstreckenden Schrittbereich 12 zusammengesetzt. Die ersten Laschen 6 enthalten jeweils die elastischen Teile 13, deren Zwischenabschnitte sich entlang dem Längsausmaß der Schrittzone 12 erstrecken. Die Vorder- und Rückenteile 10, 11 sind entlang ihren in Längsrichtung gegenüberliegenden Enden 15, 16, jeweils mit elastischen Teilen 15A, 16A ausgebildet. Diese elastischen Teile 13, 15A, 16A werden zwischen die Ober- und Unterschicht 2, 3 eingefügt und intermittierend unter Spannung an einer dieser Schichten befestigt. Die zweiten Laschen 7 enthalten in ihren Oberrändern 17 die elastischen, intermittierend unter Spannung an der Oberseite der Unterschicht 3 befestigten/verklebten elastischen Teile 18. Das Rückenteil 11 ist auf transversal gegenüberliegenden Seitenrändern jeweils mit Befestigungsbändern 20 versehen, wovon jedes an einem der Enden an dem jeweiligen Seitenrand befestigt/verklebt ist.

In einer solchen Anordnung sind die zweiten Laschen 7 innerhalb eines notwendigen Ausmaßes von 30 mm oder mehr innerhalb der in Längsrichtung gegenüberliegenden Enden 15, 16 der Vorder- und Rückenteile 10, 11 ausgebildet, und ihre Zwischenabschnitte erstrecken sich entlang der Längsausdehnung der Schrittzone 12. Jede der zweiten Laschen 7 wird von einem Oberrand 17 definiert, der in Längsrichtung die gegenüberliegenden Enden 21, 22 davon unter Kontraktion des elastischen Teiles 18 linear verbindet, und einem Bodenrand 19, der die Enden 21, 22 in einer gekrümmten Linie verbindet. Die Enden 21, 22 entsprechen im wesentlichen den Punkten, bei denen die Enden 18A, 18B der elastischen Teile 18 auf der Unterschicht 3 verbunden/verklebt sind Die Ober- und Unterschichten 2, 3 sind entlang des elastischen Teiles 18 zu der Unterseite so gefaltet, daß eine Falte mit dem Oberrand 17 gebildet wird, und daß die Innenflächen dieser Falte, d.h. einander gegenüberliegende Bereiche der Unterschicht 3, intermittierend miteinander verbunden/verklebt sind. Die zweite Lasche 7 ragt entlang des Bodenrandes 19 auf der Oberseite der Windel empor, wenn sich das elastische Teil 18 zusammenzieht, und ein Abstand zwischen dem Ober- und dem Bodenrand 17, 19 entspricht einer Projektionshöhe H der zweiten Lasche 7 in einem solchen emporragenden Zustand.

Um diese zweiten Laschen 7 zu erhalten, werden die elastischen Teile 18 unter Spannung auf die Oberseite der Unterschicht 3 verbunden/verklebt, welche nicht durchhängt, und dann werden die elastischen Teile 18 in einen geeigneten Maße zusammengezogen, so daß die Windel 1 wie ein Bogen durchhängt, wobei die elastischen Teile 18 die Bänder/Schnüre bilden. Nach einem solchen Durchhängen werden die Ober- und Unterschicht 2, 3 entlang den Gesamtlängen der jeweiligen elastischen Teile 18 gefaltet, welche sich zwischen den gegenüberliegenden Enden 18A, 18B zu der Rückseite um eine Breite h (nicht gezeigt) erstrecken, wobei die Oberränder von dem jeweiligen elastischen Teil 18 definiert werden, und die einander gegenüberliegenden Bereiche der Unterschicht 3 werden miteinander verbunden. Das Verbinden der elastischen Teile 18 mit der Unterschicht 3 und das Verbinden der Bereiche der Unterschicht 3 zueinander werden bevorzugt intermittierend durchgeführt, um das leichte Zusammenziehen der elastischen Teile 18 zu erleichtern. Die Breite h jeder Falte entspricht der Höhe H jeder zweiten Lasche 7, und eine Lage des Bodenrandes 19 relativ zu dem Oberrand 17 wird auf solche Art bestimmt, daß das Maß h in zunehmendem Maße von dem Minimum bei den gegenüberliegenden Enden 18A, 18B zu dem Maximum in der Mitte des Schrittbereiches 12 zunimmt. Auf diese Weise kann die zweite Lasche 7 auf der Oberseite der Windel 1 emporragen, wenn die Windel 1 in einer Bogenform durchhängt, und ihre Projektionshöhen können nach und nach von dem Maximum in der Mitte der Schrittzone 12 in Längsrichtung jeweils zu dem Vorder- und dem Rückenteil 10, 11 abnehmen.

Bezugnehmend auf (A) und (B) von Fig. 3 werden alternative Ausführungsbeispiele der zweiten Laschen 7, die sich von denen der Fig. 2 unterscheiden, in ähnlichen Ansichten wie in Fig. 2 dargestellt. Bezugnehmend auf (A) sind die elastischen Teile 18 zwischen der Ober- und Unterschicht 2, 3 eingelegt und intermittierend an wenigstens einer dieser Schichten 2, 3 befestigt. Bezugnehmend auf (B) ist andererseits die Oberschicht 2 relativ schmal und endet innerhalb der jeweiligen zweiten Lasche 7, d.h., die zweiten Laschen 7 sind nur durch die Unterschicht 3 ausgebildet. Im übrigen können die zweiten Laschen 7 sowohl von der Ober- als auch von der Unterschicht 2, 3 gebildet werden. Während die gegenüberliegenden Bereiche der Unterschicht 3 als sich eng berührend dargestellt sind, können diese gegenüberliegenden Bereiche in Richtung auf die Bodenränder 19 aufgrund des intermittierenden Verbindens zunehmend voneinander beabstandet sein.

Im Bezug auf Fig. 1 sind Bereiche der Ober- und der Unterschicht 2, 3, die sich über den Umfang des absorbierenden Kernes 4 nach außen erstrecken, wasserdicht miteinander um den absorbierenden Kern 4 herum verbunden. Die Oberschicht 2 kann aus nichtgewebtem Textilerzeugnis oder porösem Kunststoffilm hergestellt sein, die Unterschicht 3 kann aus einem Kunststoffilm hergestellt sein, und der absorbierende Kern 4 kann aus flockigem Faserstoff, einer Mischung von flockigem Faserstoff und superabsorbierendem Polymer oder dergleichen hergestellt sein. Verbinden und Befestigen zum Herstellen der Windel 1 kann durch die Verwendung von Klebstoff, wie Heißschmelz-Klebstoff, haftender Klebstoff, Wärmeversiegelungstechnik oder dergleichen bewirkt werden.

Gemäß der Erfindung wird eine Windel mit Laschen ausgestattet, die innerhalb eines notwendigen Ausmaßes in Längsrichtung begrenzt sind, so daß sich ihre Zwischenbereiche entlang der Schrittzone erstrecken und geeignet sind, auf der Oberseite der Windel emporzuragen, wenn sie einem Träger umgelegt wird, so daß diese Laschen ihre maximale Höhe in der Mitte der Schrittzone erreichen, in welcher sich eine Menge ausgeschiedener Körperflüssigkeiten anhäufen kann. Eine solche Anordnung löst das Problem, daß die nahe der Taillelinie liegenden Windelbereiche sperrig und steif werden, was einen Träger dazu veranlaßt, sich unwohl zu fühlen, ohne daß ein Verlust des Körperflüssigkeits-Dämmeffekts auftritt.

## Patentansprüche

1. Einwegwindel (1), die im wesentlichen eine flüssigkeitsdurchlässige Oberschicht (2), eine flüssigkeitsundurchlässige Unterschicht (3) und einen zwischen diesen Schichten angeordneten absorbierenden Kern (4) umfaßt, wobei die Oberschicht (2) und/oder die Unterschicht (3) sich über die transversal gegenüberliegenden Seitenränder des absorbierenden Kernes (4) hinaus erstrecken, und zudem elastische Teile (18) umfassen, die an dem jeweiligen Abschnitt der Oberschicht (2) und/oder der Unterschicht (3) befestigt sind, die sich nach außen über die transversal gegenüberliegenden Ränder des absorbierenden Kernes (4) entlang wenigstens eines Längsausmaßes eines Schrittbereichs der Windel (1) erstrecken, wobei die jeweiligen Abschnitte entlang der jeweiligen elastischen Teile (18) durch Falten gebildet sind, die von der Oberseite (2) der Windel (1) vorstehen und gegenüberliegende Innenseiten der jeweiligen Falten aneinander befestigt sind, wobei die Falten sich jeweils nicht über ein notwendiges Ausmaß hinauserstrecken, das in Längsrichtung innerhalb der in Längsrichtung gegenüberliegenden Enden (15, 16) der Windel (1) endet und daß diese Falten Laschen (7) bilden, die geeignet sind, auf der Oberseite der Windel (1) innerhalb des notwendigen Ausmaßes emporzuragen, welches in Längsrichtung der Windel (1) definiert ist, **dadurch gekennzeichnet,** daß die Falten 30 mm oder mehr vor den in Längsrichtung gegenüberliegenden Enden (15, 16) der Windel enden und daß sie von den in Längsrichtung gegenüberliegenden Enden (18A,18B) der jeweiligen elastischen Teile (18) zu der Mitte des Schrittbereichs (12) zunehmend breiter werden.

## Claims

1. Disposable nappy (1), which comprises essentially a liquid-permeable upper layer (2), a liquid-impermeable lower layer (3) and an absorbent core (4) arranged between these layers, the upper layer (2) and/or the lower layer (3) extending beyond the transversely opposite lateral edges of the absorbent core (4), and additionally comprising elastic parts (18) which are fastened to the respective portions of the upper layer (2) and/or the lower layer (3) which extend outwardly beyond the transversely opposite edges of the absorbent core (4) along at least a longitudinal extent of a crotch region of the nappy (1), the respective portions along the respective elastic parts (18) being formed by folds which protrude from the upper side (2) of the nappy (1), and opposite inner sides of the respective folds being fastened to one another, the folds in each case not extending beyond a necessary extent which ends, in the longitudinal direction, within the ends (15, 16) of the nappy (1) which are opposite in the longitudinal direction and these folds forming flaps (7) which are adapted to rise up on the upper side of the nappy (1) within the necessary extent which is defined in the longitudinal direction of the nappy (1), characterized in that the folds end 30 mm or more before the ends (15, 16) of the nappy which are opposite in the longitudinal direction and in that they become increasingly wider from ends (18A, 18B), which are opposite in the longitudinal direction, of the respective elastic parts (18) to the middle of the crotch region (12).

## Revendications

1. Lange à usage unique (1) comprenant, substantiellement, une couche supérieure (2) perméable aux liquides, une couche inférieure (3) imperméable aux liquides, et un corps central absorbant (4) inséré entre ces couches, la couche supérieure (2) et/ou la couche inférieure (3) s'étendant au-delà des bords latéraux transversalement opposés du corps central absorbant (4) et comprenant, en outre, des éléments élastiques (18) qui sont fixés à la partie concernée de la couche supérieure (2) et/ou de la couche inférieure (3) et s'étendent vers l'extérieur au-delà des bords transversalement opposés du corps central absorbant (4), le long d'au moins une longueur d'une zone de l'entrejambe du lange (1), des portions longeant les éléments élastiques (18) étant constituées de fronces qui s'élèvent, à partir de la face supérieure du lange (1), tandis que des faces intérieures opposées des fronces sont fixées les unes aux autres, lesdites fronces ne s étendant pas au-delà d'une mesure nécessaire laquelle se termine, dans le sens de la longueur, à l'intérieur d'une aire délimitée par les extrémités longitudinales opposées (15, 16) du lange (1), ces fronces formant des replis (7) aptes à s'élever sur la face supérieure du lange (1), dans les limites de la mesure nécessaire définie dans le sens de la longueur du lange (1), caractérisé en ce que les fronces se terminent à 30 mm ou plus des extrémités longitudinales opposées (15, 16) du lange, et en ce qu'elles deviennent de plus en plus larges, partant des extrémités longitudinales opposées (18A, 18B) des éléments élastiques (18), vers le centre de la région d'entrejambe (12).
